# EUROPÄISCHE PATENTANMELDUNG

(11) **EP 0 671 408 A2**
(43) Veröffentlichungstag der Anmeldung: **13.09.1995**
(21) Anmeldenummer: 95103160.8
(22) Anmeldetag: 06.03.1995
(51) Int. Cl.: C07H 15/207, A61K 31/70, C07H 3/04, C07H 3/06, C07H 17/04, G01N 33/569, G01N 33/574

(54) **Kohlenhydratmimetika mit antiadhäsiven Eigenschaften**

(30) Priorität: 11.03.1994 DE 4408248
(71) Anmelder: HOECHST AKTIENGESELLSCHAFT, D-65929 Frankfurt am Main (DE)
(72) Erfinder: Toepfer, Alexander, Dr., D-65719 Hofheim (DE); Kretzschmar, Gerhard, Dr., D-65760 Eschborn (DE); Bartnik, Eckart, Dr., D-65205 Wiesbaden (DE); Seiffge, Dirk, Dr., D-55246 Mainz-Kostheim (DE)

(57) **Zusammenfassung**

Die Erfindung betrifft Kohlenhydratmimetika mit antiadhäsiven Eigenschaften und im besonderen eine verbindung der allgemeinen Formel I, 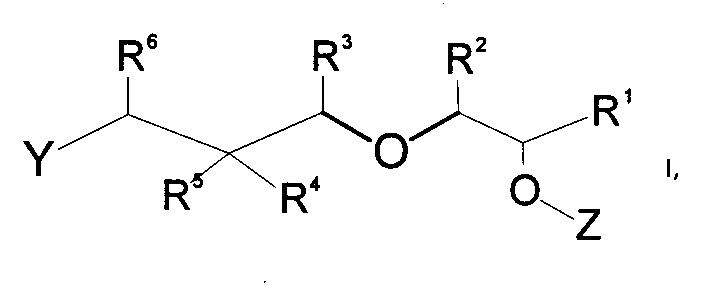
in der
- R¹ und R²: unabhängig voneinander
H, CH₂X oder CH₂O(CH₂)ₘX¹ bedeuten oder gemeinsam einen sechsgliedrigen Carbo-oder Heterocyclus mit mindestens einem der Substituenten R⁷, R⁸ und R⁹ bilden und
- R³: O, S, H oder -CH₂OX² bedeutet und
- R⁴ und R⁵: unabhängig voneinander
O-α-NANA, O-β-NANA, O(CR¹⁰R¹¹)ₙCOOH, OCX³₂(CR¹⁰R¹¹)ₙCOOH, OSO₃H oder eine anderer einbasiger Säurerest bedeuten und
- R⁶: H, -OH oder C₁-C₂₅-Alkyl bedeutet oder mit R³ einen sechsgliedrigen Carbo- oder Heterocyclus mit mindestens einem Substituenten X⁴ bildet und
- Y: O-α-NANA, O-β-NANA, O(CR¹⁰R¹¹)ₚCOOH, OCX³₂(CR¹⁰R¹¹)ₚCOOH, OSO₃H oder eine andere einbasige Säure bedeutet und
- Z: eine Pyranose, eine Furanose oder einen offenkettigen Polyalkohol darstellt und
- m, n, p und q: unabhängig voneinander eine Zahl von 1 bis 20 und
- R⁷, R^{8,} R⁹, X, X^{1,} X² und X⁴: unabhängig voneinander H, -NH₂, - COOH, -OH, - CH₂OH, -CH₂NH, C₁-C₂₅-Alkyl, Aryl, oder - CH₂O(CH₂)_{q}X,
- X³: H, C₁-C₂₅-Alkyl oder Aryl bedeuten oder wahlweise
- X³₂: für =O oder =S steht und
- R¹⁰ und R¹¹: unabhängig voneinander X oder - CH₂X bedeuten oder gemeinsam einen sechsgliedrigen Carbo-oder Heterocyclus mit mindestens einem Substituenten X⁴ bilden,
ausgenommen die Verbindungen Sialyl Lewis-X und -A sowie deren Derivate, welche anstelle einer N-Acetylgruppe die Substituenten N₃, NH₂ oder OH oder welche anstelle von Fucose Glycerin tragen,
ferner ein Verfahren zur Herstellung dieser Verbindung, deren Verwendung sowie aus dieser hergestellte Arzneimittel und Diagnostika.

## Beschreibung

Die Erfindung betrifft Kohlenhydratmimetika mit antiadhäsiven Eigenschaften, ein Verfahren zu deren Herstellung, deren Verwendung sowie aus diesen hergestellte Arzneimittel und Diagnostika.

Die auf der Oberfläche eukaryontischer Zellen befindlichen Glycoconjugate stellen spezifische Bindungsepitope für Selektine (= transmembranale Proteine) dar. Diese Selektine, welche sowohl im Endothel als auch auf den verschiedenen zirkulierenden Zellen des hämatolymphoiden Systems vorkommen, gehen spezifische Wechselwirkungen mit Kohlenhydrat-Epitopen (Liganden) des jeweils anderen Zelltyps ein (K.-A. Karlsson, TIPS 12, (1991), 265-272). Synthetische Analoga der Selektin-Liganden werden als vielversprechende Verbindungen für Antiphlogistika und Antikoagulantien angesehen (T. A. Springer, L.A. Lasky, Nature 349, 1991, 196-197; T. Feizi, TIBS 16, 1991, 84-86).

Kohlenhydratliganden spielen außerdem als Erkennungsdomänen für Viren (J. C. Paulson, The Receptors, Vol. II, Ed.: P. M. Conn, Academic press, 1985, 131), Bakterien (Strömberg et al. EMBO J. 1990, (9), 2001) und Toxine (Karlsson et al. Sourcebook of Bacterial Protein Toxins, Eds. J. E. Alouf, J. H. Freer, Academic Press, 1990, (56), 3537) eine entscheidende Rolle und damit auch bei der Prophylaxe, Diagnostik oder Therapie von bakteriellen und viralen Infektionen, entzündlichen Erkrankungen, rheumatischer Arthritis, Allergien, Nachinfarktsyndrom, Schock, Schlaganfall, akute und chronische Organabstoßung, Vasculitis, Sepsis, Schocklunge, u.s.w..

Von besonderer Bedeutung bei der Einleitung der oben genannten Erkrankungen sind sialylierte und/oder fucosylierte Kohlenhydrate (Sialic Acids in "Cell Biology Monographs" Schauer, Editor, Vol. 10, 1982). Besonders als Mediatoren der Zelladhäsion spielen diese eine entscheidende Rolle (Lowe et al., Cell, 63, 475-485, 1990). Eine besondere Bedeutung kommt in diesem Zusammenhang Sialyl-Lewis-X [αNeu5Ac(2→3)βGal(1→4)[αFuc(1→3)]-βGlcNAc-OR] und Sialyl-Lewis-A [αNeu5Ac(2→3)βGal(1→3)[αFuc(1→4]-βGlcNAc-OR] zu (R ist definiert als ein Aglycon mit mindestens einem Kohlenstoffatom). Für diese Verbindungen wurden sowohl chemische (Ratcliffe et al., U.S. Patent No. 5,079,353) als auch chemisch/enzymatische Synthesen entwickelt (A. Venot et al., PCT/CA 92/00251).

Mit dem Ziel einer Vereinfachung der sehr aufwendigen Synthesen dieser Verbindungen, bei Erhaltung oder Verbesserung ihrer Bindungsaffinitäten zu den entsprechenden Selektinen, wurden bereits mehrere Strukturen vorgeschlagen und teilweise synthetisiert. So wurde beispielsweise Neuraminsäure durch Milch- oder Glycolsäure und/oder Fucose durch Glycerin bzw. Trifluormethyl-Fucose und/oder N-Acetylglucosamin durch Glucosamin oder Glucose ersetzt (PCT/US92/09870). Eine Substitution von Neuraminsäure durch Sulfat oder Phosphat ist ebenfalls beschrieben (PCT/CA 92/00245). Beschrieben ist außerdem eine Substitution von Glucosamin durch eine Kette von mindestens 2 Kohlenstoffatomen (WO 92/18610). Nach dem heutigen Stand der Technik ist lediglich für das native Sialyl-Lewis-X und Sialyl-Lewis-A mit geringfügigen Modifikationen eine effiziente, enzymatische "Large Scale-Synthese" entwickelt worden (C.H. Wong et al., WO 92/16640 und US 5,162,513). Diese Verbindungen haben jedoch den Nachteil einer geringen Affinität zu den Selektinen zum einen und einer geringen in vivo-Stabilität zum anderen (Wirkstoffe sind nicht oral verfügbar).

Für eine chemische Synthese von Sialyl-Lewis-X und Derivaten existieren bis dato nur extrem vielstufige und daher wenig praktikable Synthesen. Für Verbindungen, die statt des anomeren Zuckers ein Diol oder eine ähnliche Gruppe tragen, wurden bisher noch keine Synthesen beschrieben.

In WO 92/18610 werden zwar Stukturen vorgeschlagen, bei welchen Glucosamin durch diverse cyclische Diole substituiert werden könnte. Es ist nicht beschrieben, ob und wie sich aus diesen Verbindungen oligo- bzw. polyvalente Strukturen synthetisieren lassen. In allen Publikationen findet die dafür nötige Verknüpfung über die anomere Position des am reduzierenden Ende befindlichen Zuckers statt, der in WO 92/18610 substituiert werden soll und damit für diesen Zweck nicht mehr zur Verfügung steht.

Demgegenüber ist es Aufgabe der vorliegenden Erfindung, neue, physiologisch stabilere Selektin-Liganden bereitzustellen, welche eine gegenüber den natürlichen Liganden vergleichbare oder stärkere Wechselwirkung mit dem Rezeptor aufweisen und sich im Vergleich zu den natürlichen Liganden leicht herstellen lassen.

Es ist ferner Aufgabe der vorliegenden Erfindung, auf Basis dieser neuen Selektin-Liganden Arzneimittel zur Therapie oder Prophylaxe und Mittel zur Diagnose von Krankheiten verfügbar zu machen, bei welchen bakterielle oder virale Infektionen, metastasierende Tumore oder entzündliche Prozesse in volviert sind.

Die Aufgabe wird erfindungsgemäß gelöst durch
1. eine Verbindung der allgemeinen Formel I 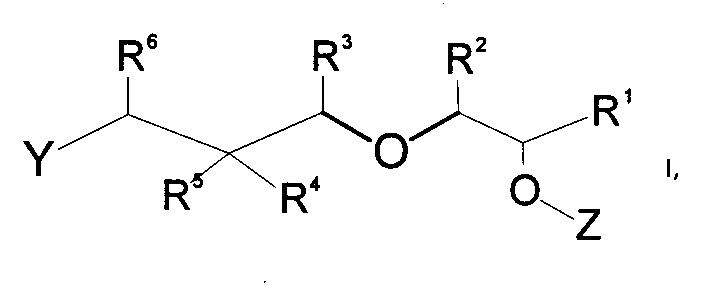 in der
   - R¹ und R²: unabhängig voneinander
   H, CH₂X oder CH₂O(CH₂)ₘX¹ bedeuten oder gemeinsam einen sechsgliedrigen Carbo- oder Heterocyclus mit mindestens einem der Substituenten R⁷, R⁸ und R⁹ bilden und
   - R³: O, S, H oder -CH₂OX² bedeutet und
   - R⁴ und R⁵: unabhängig voneinander
   O-α-N-Acetylneuraminsäure (O-α-N-NANA),
   O-β-N-Acetylneuraminsäure (O-β-N-NANA),
   O(CR¹⁰R¹¹)ₙCOOH, OCX³₂(CR¹⁰R¹¹)ₙCOOH, OSO₃H oder eine andere einbasige Säure bedeuten und
   - R⁶: H, -OH oder C₁-C₂₅-Alkyl bedeutet oder
   mit R³ einen sechsgliedrigen Carbo- oder Heterocyclus mit mindestens einem Substituenten X⁴ bildet und
   - Y: O-α-N-Acetylneuraminsäure (O-α-N-NANA), O-β-N-Acetylneuraminsäure (O-β-N-NANA),
   O(CR¹⁰R¹¹)ₚCOOH, OCX³₂(CR¹⁰R¹¹)ₚCOOH, OSO₃H oder eine andere einbasige Säure bedeutet und
   - Z: eine Pyranose, eine Furanose oder einen offenkettigen Polyalkohol darstellt und
   - m, n, p und q: unabhängig voneinander eine Zahl von 1 bis 20 und
   - R⁷, R⁸, R⁹, X, X¹, X² und X⁴: unabhängig voneinander H, -NH₂, -COOH, -OH, -CH₂OH, -CH₂NH, C₁-C₂₅-Alkyl, Aryl, oder -CH₂O(CH₂)_{q}X,
   - X³: H, C₁-C₂₅-Alkyl oder Aryl bedeuten oder wahlweise
   - X³₂: für =O oder =S steht und
   - R¹⁰ und R¹¹: unabhängig voneinander X oder -CH₂X bedeuten oder gemeinsam einen sechsgliedrigen Carbo- oder Heterocyclus mit mindestens einem Substituenten X⁴ bilden,
   ausgenommen die Verbindungen Sialyl-Lewis-X und -A sowie deren Derivate, welche anstelle einer N-Acetylgruppe die Substituenten N₃, NH₂ oder OH oder welche anstelle von Fucose Glycerin tragen,
2. insbesondere durch eine Verbindung der allgemeinen Formel I, welche sich dadurch auszeichnet, daß R³ und R⁶ gemeinsam einen β-D-Galactosylrest bilden,
3. vorzugsweise, dadurch gekennzeichnet, daß
   Z ein O-α-Fucopyranosylrest ist.
4. Vorzugsweise wird die Aufgabe durch eine Verbindung der allgemeinen Formel I mit den kennzeichnenden Merkmalen gemäß Nr. 2 und 3 gelöst, welche sich dadurch auszeichnet, daß
   - Y: ein O-α-N-Acetylneuraminsäurerest ist und
   - R¹ und R²: H bedeuten, nämlich 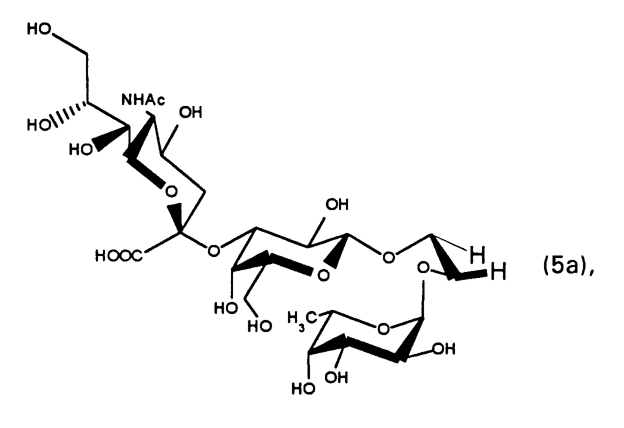
5. oder welche sich dadurch auszeichnet, daß
   - Y: ein O-α-N-Acetylneuraminsäurerest ist und
   - R¹ und R²: -CH₂O(CH₂)₅CH₃ bedeuten, nämlich 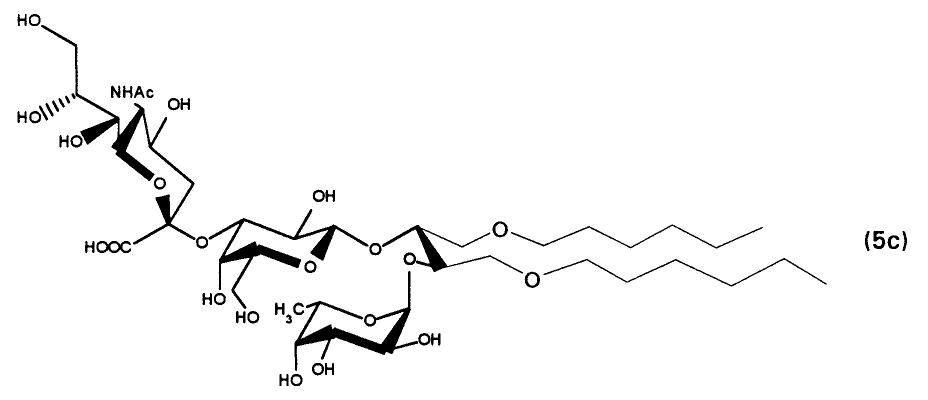
6. oder vorzugsweise durch eine Verbindung der allgemeinen Formel I mit den kennzeichnenden Merkmalen gemäß Nr. 2 und 3, welche sich
   dadurch auszeichnet,daß
   R¹ und R² gemeinsam einen sechsgliedrigen Carbocyclus bilden und die Substituenten R⁷, R⁸ und R⁹ H bedeuten,
7. insbesondere dadurch gekennzeichnet, daß
   Y ein O-α-N-Acetylneuraminsäurerest ist, nämlich 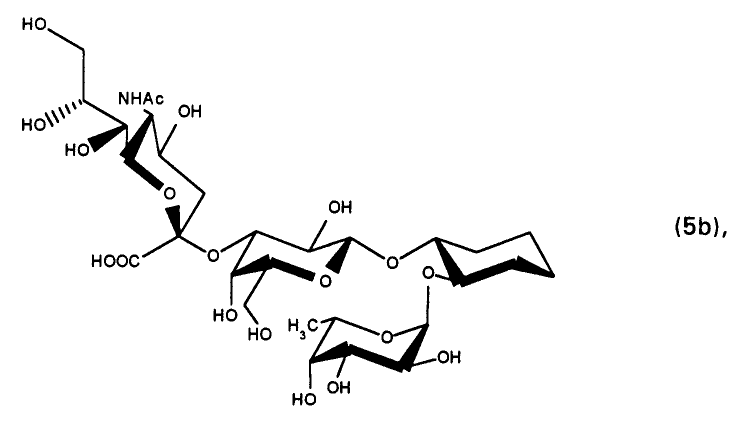
8. oder dadurch gekennzeichnet, daß
   Y HOOC-CH₂- bedeutet, nämlich 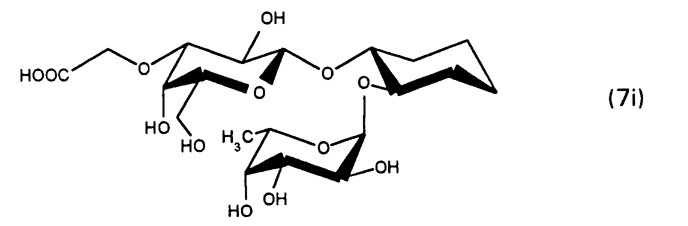
9. oder durch eine Verbindung mit den unter Nr. 2 dargestellten Merkmalen, welche sich ferner dadurch auszeichnet, daß
   R¹ und R² gemeinsam einen sechsgliedrigen Carbocyclus bilden und die Substituenten R⁷, R⁸ und R⁹ H bedeuten, Y ein O-α-N-Acetylneuraminsäurerest ist und Z ein α-Mannosylrest ist, nämlich 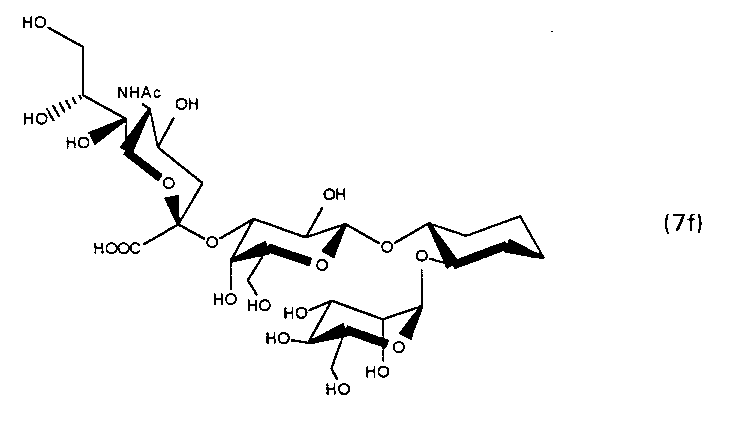
10. oder durch eine Verbindung mit den unter Nr. 2 dargestellten Merkmalen, welche sich ferner dadurch auszeichnet, daß R¹ und R² gemeinsam einen sechsgliedrigen Carbocyclus bilden und die Substituenten R⁷, R⁸ und R⁹ H bedeuten, Y ein O-α-N-Acetylneuraminsäurerest ist und Z ein α-Glucosylrest ist, nämlich 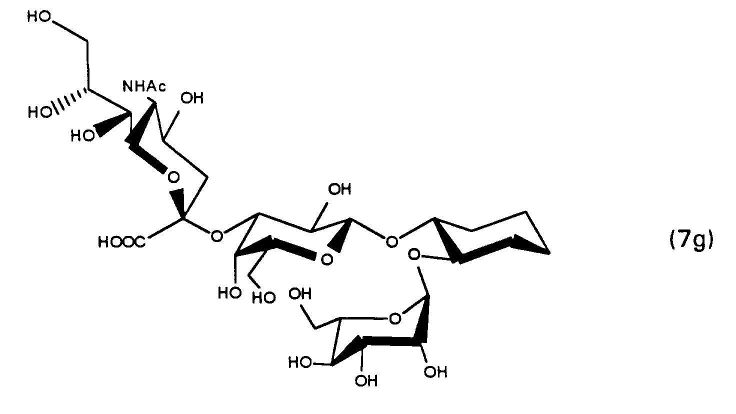
11. oder durch eine Verbindung mit den unter Nr. 2 dargestellten Merkmalen, welche sich ferner dadurch auszeichnet, daß
   R¹ und R² gemeinsam einen sechsgliedrigen Carbocyclus bilden und die Substituenten R⁷, R⁸ und R⁹ H bedeuten und Z -CH₂C(CH₂OH)₃ bedeutet,
12. vorzugsweise dadurch gekennzeichnet, daß
   Y ein α-N-Acetylneuraminsäurerest ist, nämlich 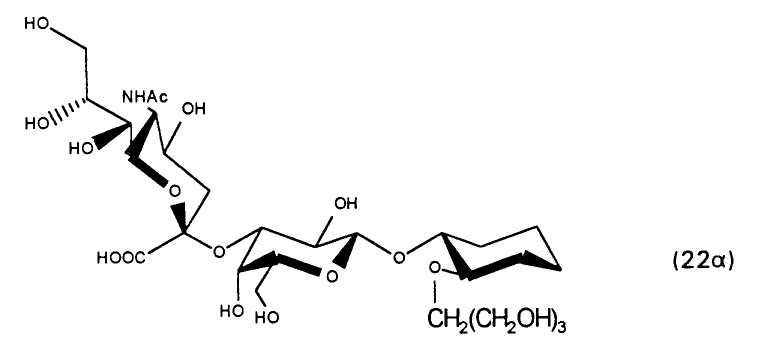
13. oder vorzugsweise dadurch gekennzeichnet, daß
   Y ein O-β-N-Acetylneuraminsäurerest ist, nämlich 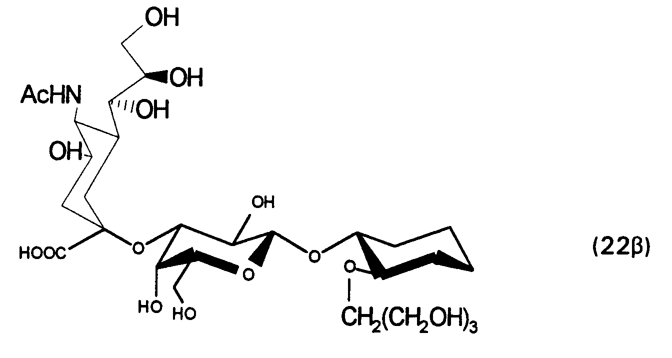
14. oder durch eine Verbindung mit den unter Nr. 3 dargestellten Merkmalen, welche sich ferner dadurch auszeichnet, daß
   R¹ und R² gemeinsam einen sechsgliedrigen Carbocyclus bilden und die Substituenten R⁷, R⁸ und R⁹ H bedeuten, Y ein α-N-Acetylneuraminsäurerest ist und der β-D-Galactosylrest in 2-O-Position mit einer Hexylgruppe substituiert ist, nämlich 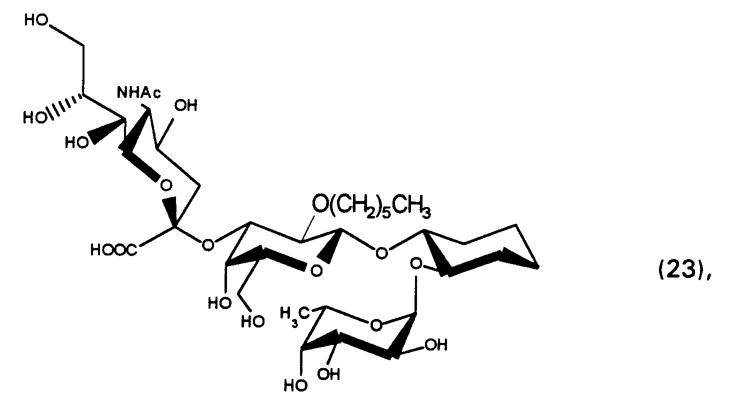
15. oder durch eine Verbindung der Formel I gemäß Nr. 1, welche sich dadurch auszeichnet, daß
   R¹ und R² gemeinsam einen sechsgliedrigen Carbocyclus bilden, R³, R⁶, R⁷, R⁸ und R⁹ H bedeuten und
   Z ein α-Fucopyranosylrest ist,
16. vorzugsweise, dadurch gekennzeichnet, daß
   R⁴ H und R5 -OH bedeuten,
17. insbesondere, dadurch gekennzeichnet, daß
   Y α-O-N-Acetylneuraminsäure bedeutet, nämlich 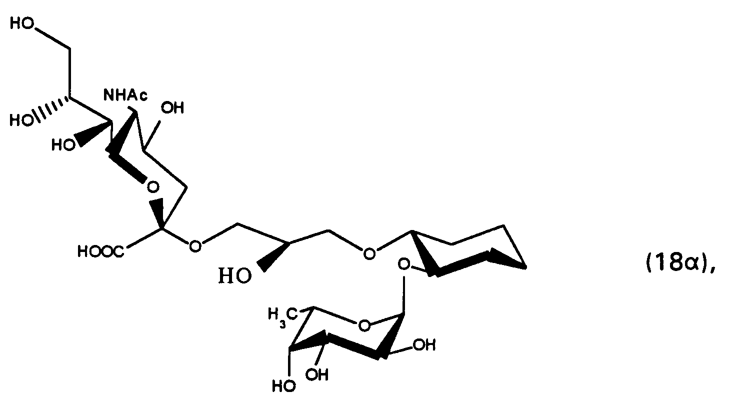
18. oder, dadurch gekennzeichnet, daß
   Y O-β-N-Acetylneuraminsäure bedeutet, nämlich 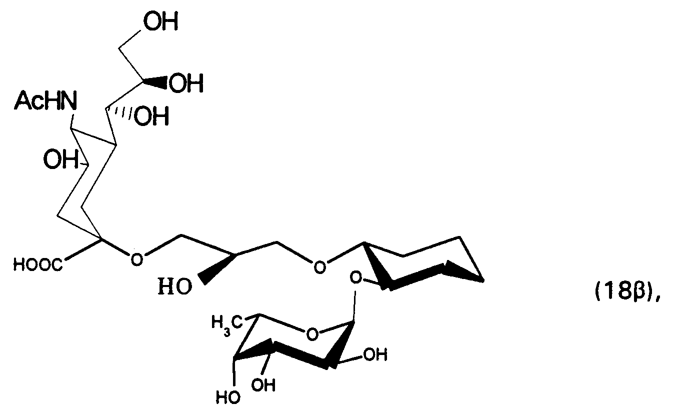
19. oder durch eine Verbindung mit den unter Nr. 15 bezeichneten Merkmalen, welche sich dadurch auszeichnet, daß R⁴ und R5 H bedeuten,
20. vorzugsweise, dadurch gekennzeichnet, daß
   Y O-α-N-Acetylneuraminsäure bedeutet, nämlich 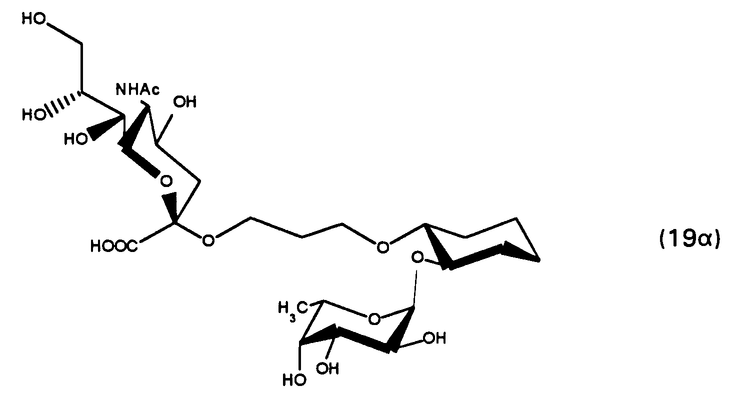
21. oder dadurch gekennzeichnet, daß
   Y O-β-N-Acetylneuraminsäure bedeutet, nämlich 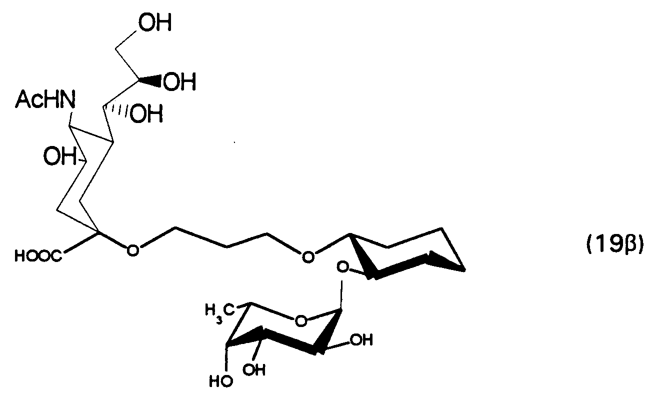
22. oder durch eine Verbindung mit den Merkmalen gemäß Nr. 15, welche sich dadurch auszeichnet, daß
   R⁴ und R⁵ -CH₂OH bedeuten,
23. vorzugsweise dadurch gekennzeichnet, daß
   Y O-α-N-Acetylneuraminsäure bedeutet, nämlich 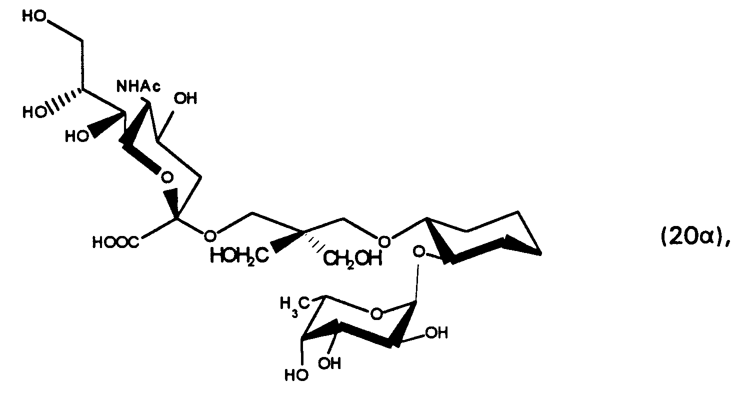
24. oder dadurch gekennzeichnet, daß
   Y O-β-N-Acetylneuraminsäure bedeutet, nämlich 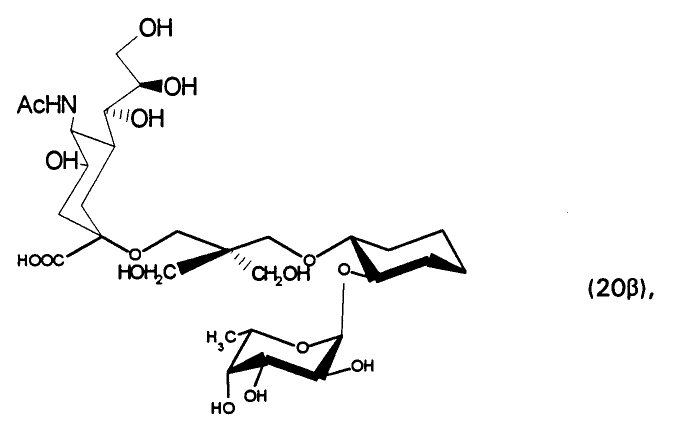
25. oder durch eine Verbindung der Formel I mit den Merkmalen gemäß
   Nr. 1, welche sich dadurch auszeichnet, daß
   R¹ und R² gemeinsam einen substituierten Tetrahydropyranring bilden und
   - R⁷ und R⁸: H und
   - R⁹: -CH₂O(CH₂)_{q}X bedeuten,
   - Z: ein α-Fucopyranosylrest ist
   - R³ und R⁶: gemeinsam einen β-D-Galactosylrest bilden und
   - Y: ein O-α-N-Acetylneuraminsäurerest ist,
26. vorzugsweise dadurch gekennzeichnet, daß
   R⁹ -CH₂O(CH₂)₅CH₃ bedeutet, nämlich (7h), 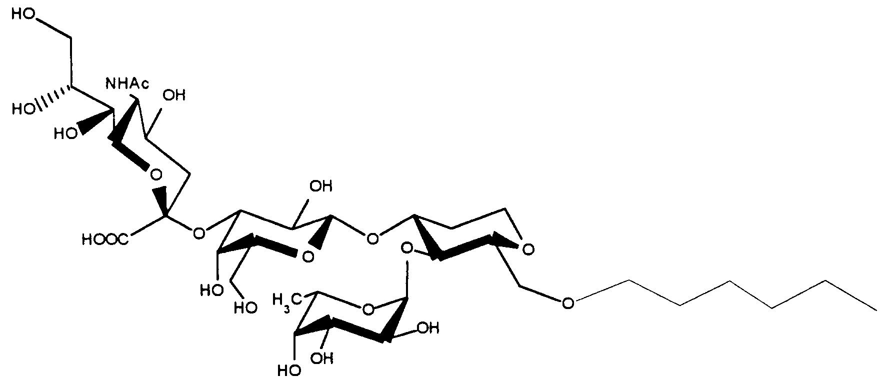
27. oder dadurch gekennzeichnet, daß
   R⁹ -CH₂OH bedeutet, nämlich 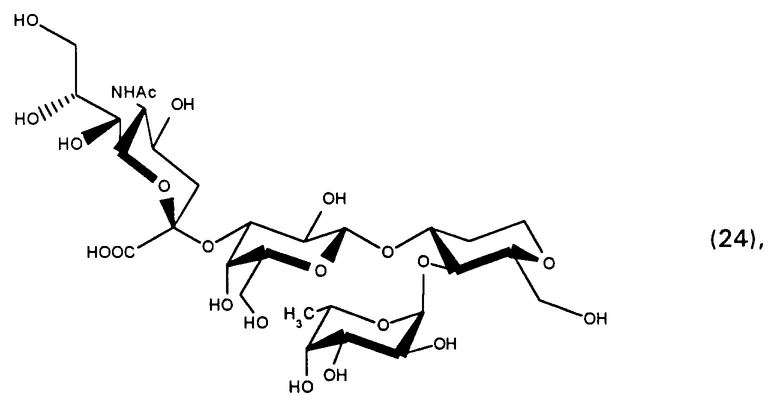
28. oder dadurch gekennzeichnet, daß
   R⁹ -CH₂O(CH₂)₃C₆H₅ bedeutet, nämlich (7e). 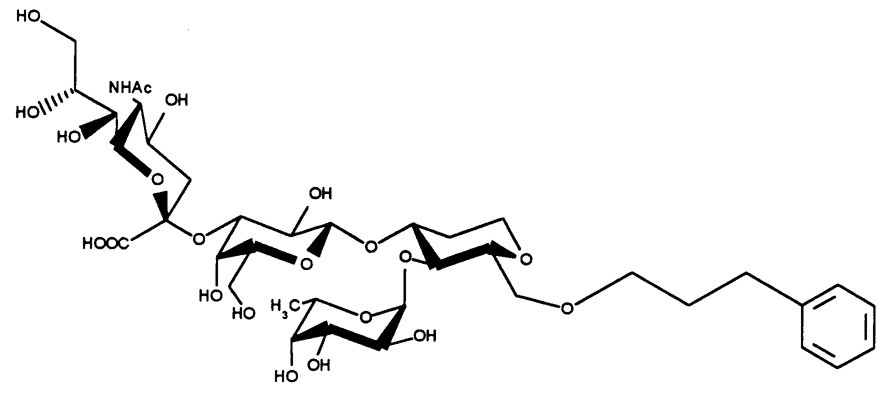
29. Die eingangs gestellte Aufgabe wird ferner gelöst durch ein Verfahren zur Herstellung einer Verbindung der Formel I, welches dadurch gekennzeichnet ist, daß man zunächst unter Alkylierung oder Glycosylierung einer funktionellen Gruppe eines Akzeptors II; 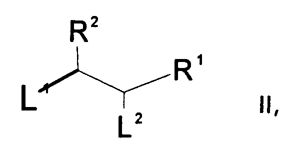 aufweisend mindestens zwei benachbarte funktionelle Gruppen L¹ und L² sowie aufweisend die Substituenten R¹ und R², mittels eines Äquivalentes eines mindestens zwei funktionelle Gruppen L³ und L⁴ tragenden Donors III, 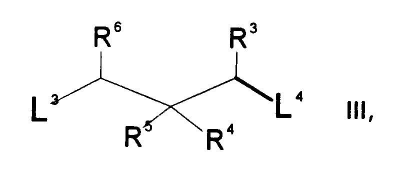 dessen eine funktionelle Gruppe L³ notwendigenfalls geschützt und dessen andere funktionelle Gruppe L⁴ gebenenfalls aktiviert vorliegt, Zwischenverbindung IV, 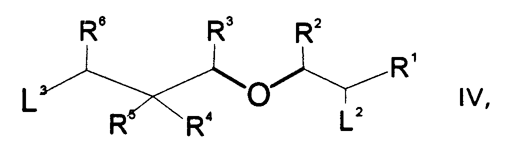 herstellt, wonach man unter Alkylierung, Acylierung oder Glycosylierung der ungeschützten funktionellen Gruppe L² der Zwischenverbindung IV mittels eines mit einer aktivierten funktionellen Gruppe L⁵ versehenen Donors V,

   Z-L⁵ V,

   dessen übrige funktionelle Gruppen notwendigenfalls Schutzgruppen tragen, Zwischenverbindung VI, 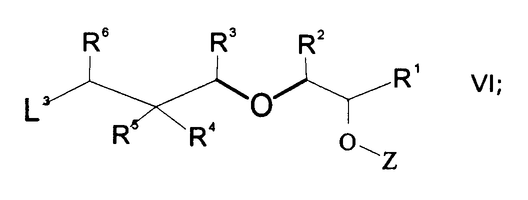 herstellt und schließlich gegebenenfalls nach selektiver Entschützung und Aktivierung der funktionellen Gruppe L³ durch Umsetzung der Zwischenverbindung VI mit Donor VII,

   Y-L⁶ VII,

   der mit einer aktivierten funktionellen Gruppe L⁶ versehenen und im übrigen mit Schutzgruppen versehen ist, und nachfolgender Abspaltung sämtlicher Schutzgruppen die Verbindung der Formel I erhält, wobei sämtliche übrige Variablen die unter Nr.1 genannten Bedeutungen haben.
30. Wahlweise kann Akzeptor II zunächst mit Donor V und anschließend mit Donor III zur Zwischenverbindung VI umgesetzt werden.
31. Die Aufgabe wird ferner gelöst durch ein Arzneimittel, enthaltend eine Verbindung der allgemeinen Formel I und gegebenfalls pharmazeutische Träger.
32. Die Verbindung der allgemeinen Formel I ist vorzugsweise zur Herstellung eines Arzneimittels zur Therapie oder Prophylaxe von Krankheiten geeignet, bei welchen bakterielle oder virale Infektionen, entzündliche Prozesse oder metastasierende Tumoren involviert sind.
33. Die Verbindung der allgemeinen Formel I ist auch zur Herstellung eines Mittels zur Diagnose von Krankheiten geeignet, bei welchen bakterielle oder virale Infektionen, entzündliche Prozesse oder metastasierende Tumoren involviert sind.

Die Erfindung wird im folgenden detailliert beschrieben, insbesondere in ihren bevorzugten Ausführungsformen.

Als "Mimetika" werden Verbindungen definiert, bei welchen man, ausgehend von einem Oligosaccharid, vorzugsweise Sialyl Lewis-X oder -A
- ein oder mehrere Zucker X austauscht, die natürlicherweise nicht vorkommen. Dieser Austausch kann auch zu einer Vereinfachung der Struktur führen;
- ein oder mehrere Zucker von durch eine chemische Gruppe oder Verbindung, die nicht zu den Zuckern zählt, ausgetauscht werden. Bei diesen Verbindungen handelt es sich vorzugsweise um Di- oder Polyole. Auch dieser Austausch kann zu einer Vereinfachung der ursprünglichen Struktur führen;
- die α-Konfiguration eines in dem Oligosaccharid vorkommenden Zuckers wird durch die β-Konfiguration des Zuckers ersetzt, also die Stereochemie am anomeren Zentrum verändert.

Die erfindungsgemäßen Kohlenhydrat-Mimetika ermöglichen eine Synthese, die vereinfacht, verbilligt und gleichzeitig zu weniger glyosidischen Bindungen führt, was wiederum eine höhere physiologische Stabilität der Produkte zur Folge hat. Außerdem können die erfindungsgemäßen Mimetika höhere Affinitäten zu den natürlichen Rezeptoren, beispielsweise zu E- und/oder P-Selektin, als Sialyl Lewis-X haben.

Werden Zuckerbausteine durch einfache Alkohole ersetzt, so reduziert sich zum einen der Syntheseaufwand beträchtlich, zum anderen können die Mimetika auch noch eine höhere Affinität zu den verschiedenen Glycolipid-/Glycoprotein-Rezeptoren aufweisen. Dies soll am Beispiel der Substitution von N-Acetyl-D-glucosamin durch (1R, 2R)-trans-1,2-Cyclohexandiol erläutert werden. Diese chirale Verbindung hat nur 2 Hydroxylgruppen (N-Acetyl-D-Glucosamin: 4). Aufgrund der C2-Symmetrie dieses Diols sind beide Hydroxylgruppen äquivalent. Dies hat zur Folge, daß dieser Baustein ohne Schutzgruppen eingesetzt werden kann, da es keine Rolle spielt, welche der beiden Hydroxylgruppen zuerst verknüpft wird. Das so erhaltene Produkt kann direkt (ohne Schutzgruppenmodifikation) für die nächste Verknüpfung eingesetzt werden. Aufgrund der reduzierten Anzahl der Sauerstoffatome weisen die Hydroxylgruppen dieses Diols eine wesentlich höhere Reaktivität auf als die des N-Acetyl-D-Glucosamins, was zusätzlich zur verringerten Stufenzahl und zu höheren Ausbeuten führt.

Es sei ausdrücklich darauf hingewiesen, daß die beiden Hydroxylgruppen von (1R,2R)-trans-1,2-Cyclohexandiol die gleiche Stereochemie aufweisen wie die 3-und 4-Hydroxylgruppe von N-Acetyl-D-glucosamin. Tatsächlich weist das so erhaltene Kohlenhydrat-Mimetikum eine dreifach erhöhte Affinität sowohl zu E- als auch P-Selectin auf als Sialyl Lewis-X. Dies zeigt außerdem ganz deutlich, daß sowohl die N-Acetylgruppe in 2-Position als auch die übrigen Hydroxylgruppen für die Selektinbindung überflüssig oder gar störend sind.

Dies gilt in ähnlicher Weise für alle in dieser Erfindung vorgestellten Mimetika. Ein weiterer wichtiger Aspekt des Wertes der hier beschriebenen Derivate liegt darin begründet, daß es sich bei glycosidischen Bindungen um Acetale handelt, welche sowohl durch Säure als auch durch Glycosidasen gespalten werden können.

Wird nun, wie hier beschrieben, eine glycosidische Bindung durch eine Etherbindung ersetzt, so führt dies zu einer erhöhten Stabilität dieser Mimetika, was im Hinblick auf ein möglichst oral verabreichbares Medikament einen entscheidenden Vorteil darstellt. Wird anstatt einer glycosidischen Bindung eine Etherbindung geknüpft, entfällt im Falle primärer Alkohole außerdem die Problematik einer stereoselektiven Verknüpfung. Die Etherbindung aus dem Alkohol und aktivierter Gruppe wird in einem Zweiphasensystem, wie z. B. in Anwesenheit von Toluol und wäßriger Natronlauge, unter Phasentransferbedingungen geknüpft. Vorzugsweise geschieht dies unter Zugabe von Alkylcarbonaten und Kronether und Deprotonierung des Alkohols mit einer starken Base in einem aprotischen Lösemittel, wie z. B. DMF, DMSO, CH₂Cl₂. Als Beispiel sei die Substitution eines Zuckers durch Pentaerythrit oder Glycerin genannt. Wird beispielsweise D-Galactose durch Glycerin ersetzt, und die verbleibende primäre Hydroxylgruppe sialyliert, so zeigt sich überraschenderweise, daß das unnatürliche β-Sialosid (18β) eine höhere Bindungsaffinität sowohl zu E- als auch P-Selektin aufweist als das α-Sialosid (18α).

Dieser Sachverhalt zeigt sich auch beim Vergleich der Verbindungen 18α und 18β. Daß α-Sialoside von Sialidasen nicht gespalten werden können und auch gegenüber verdünnter Mineralsäure deutlich stabiler sind (Kuhn, Lutz, MacDonald, Chem. Ber. 611-617, 1966) ist dies ein im Vergleich zu den nativen α-Sialosiden gleich in doppelter Hinsicht ein Vorteil.

### Synthese einer Verbindung der Formel I

Die Verbindungen dieser Erfindung mit der allgemeinen Formel I lassen sich ausgehend von käuflichen Komponenten mit mindestens 2 benachbarten freien Hydroxylgruppen wie z. B. Ethylenglykol, Glycerin, (1R,2R)-trans-1,2-Cyclohexandiol (Fluka) oder beispielsweise einem D-Threitolderivat mit geeigneten Resten an den beiden primären Hydroxylgruppen (z. B. Verbindung 7) synthetisieren. Im letzgenannten Beispiel wird käufliches (-)-2,3-O-Isopropyliden-D-threitol dialkyliert und anschließend deisopropylideniert. Die Umsetzung dieser Verbindungen mit höchstens einem Äquivalent eines Gylcosyldonors (z. B. Tetraacetylgalactosyl-Trichoracetimidat in einem aprotischen Lösungsmittel unter Säurekatalyse; R. R. Schmidt, Angew. Chem. 98 (1986) 213-236), eines Dicarbonsäuremonoesters (z. B. Adipinsäuremonomethylester mit DCC und DMAP in Dichlormethan) oder eines Spacer-Triflats (z. B. das Triflat von monoallyliertem Propandiol in Dichlormethan mit Kaliumcarbonat und Kronenether oder unter Phasentransferbedingungen mit Tetrabutylammoniumbromid und Natronlauge) liefert das monoglycosilierte bzw. monoacylierte oder monoalkylierte Produkt in hohen Ausbeuten. Insbesondere können die Verbindungen der Formel I mittels aktivierter Gruppen, wie Triflate, Tosylate, Mesylate, Bromide, Jodide, Chloride, Aktivester, Carbonsäurechloride, Carbonsäureimidazolide und/oder Carbonsäureanhydride hergestellt werden. Im folgenden wird die zweite Hydroxylgruppe glycosyliert (z. B. mit perbenzylierte Gluconsäure) oder alkyliert (z. B. mit dem Monotriflat von perbenzyliertem Dulcit). Das so erhaltene vollgeschützte, disubstituierte Diol (oder Polyol) wird nach Abspaltung der Allylschutzgruppe oder der Acetylgruppen mit einer sauren Funktion versehen. Dies kann z. B. wie bei vielen natürlichen Oligosacchariden N-Acetylneuraminsäure aber beispielsweise auch Milch-, Glycol-, Glycerin- oder Zitronensäure sein. Nach Abspaltung aller Schutzgruppen erhält man die biologisch aktiven Verbindungen der Formel I.

Werden die erfindungsgemäßen Verbindungen als Anti-Adhäsionstherapeutika eingesetzt, so sollen sie im Fall von Entzündungen verhindern, daß die ELAM-1-Rezeptoren auf stimulierten Endothelzellen an Sialyl Lewis-X-Strukturen auf der Oberfläche von Leukozyten binden. Im Fall der Influenza-Therapie verhindern die Rezeptorblocker die Anheftung von Viren an die Neuraminsäure auf der Zelloberfläche und damit auch die Endozytose der Viruspartikel.

Bei den Rezeptoren, welche die Kohlenhydratmimetika erkennen können, handelt es sich vorzugsweise um solche, die an der Zelloberfläche exprimiert werden, z. B. von Säugerzellen, einschl. menschlicher Zellen, Bakterienzellen oder Viren. Auch bevorzugt sind Rezeptoren, die Hormone oder Toxine erkennen.

Besonders bevorzugt sind solche Zelloberflächenrezeptoren, die zur Klasse der Selektine gehören.

Ganz besonders bevorzugt werden die bei entzündlichen Erkrankungen exprimierten Rezeptoren, beispielsweise Leu-8 (= L-Selektin = gp90^{mel} = LAM-1 = LEC-CAM-1), ELAM-1 (= E-Selektin) und GMP-140 (= P-Selektin= CD62 = PADGEM).

Die vorliegende Erfindung befaßt sich mit der Synthese komplexer Kohlenhydrat-Mimetika, bei welchen N-Acetylglucosamin durch R-CHOH-CHOH-R ersetzt ist, wobei die stereochemische Anordnung der Hydroxylgruppen, im Falle chiraler Verbindungen, der von N-Acetylglucosamin entspricht (z. B. Ethylenglycol, Glycerin, (1R, 2R)-trans-1,2-Cyclohexandiol, D-Threitol, 1,2-Didesoxyglucose u.s.w.). Zusätzlich kann Galactose durch einen Spacer geeigneter Länge oder eine offenkettige Verbindung mit geeigneten funktionellen Gruppen ersetzt sein (z. B. Propandiol, Glycerin mit freier oder substituierter sekundärer OH-Gruppe, Pentaerythrit mit freien oder substituierten Hydroxylgruppen). Weiterhin kann α-glycosidisch verknüpfte Neuraminsäure durch β-glycosidisch verknüpfte oder durch andere geeignete Säuren wie Chinasäure, Zitronensäure, Glycolsäure, Milchsäure u.s.w. ersetzt sein. Auch die Substitution der αNANA-(2-3)-βGal-Einheit durch einen geeigneten Rest (z. B. Schleimsäure) ist möglich. Fucose kann durch andere Zucker wie Arabinose, Rhamnose, D-Galactose aber auch durch Glucit, Mannit, Pentaerythrit u.s.w. ersetzt werden.

Vorteile dieser Erfindung sind:
· Die Synthese ist im Vergleich zu dem der bisher bekannten Rezeptorblocker deutlich einfacher durchführbar; deutlich einfacher durchführbar;
· Die Synthese ist kostengünstiger;
· Die erfindungsgemäßen Rezeptorblocker sind keine komplexen Moleküle, im Vergleich zu den aus dem Stand der Technik bekannten Rezeptorblockern;
· Sie besitzen eine geringere Anzahl von glykosidischen Bedingungen und
· haben teilweise eine höhere Affinität zu E- und/oder P-Selektin als das bekannte Sialyl-Lewis-X Molekül.

Die Verbindungen gemäß der vorliegenden Erfindung stellen allesamt potentielle Liganden für die bisher bekannten Selektine (Proteine, welche auf dem Endothel und anderen Zelloberflächen exprimiert und an spezifische Kohlenhydrat-Liganden binden) dar.

### Leukozyten-Adhäsion

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. am Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung von Entzündungsmediatoren (Leukotriene, PAF) und Zytokinen (TNF-alpha, Interleukinen) zur zeitlich gestuften, massiven Expression von eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune System. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301-314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulating polymorphnuclear leukocytes to blood vessel walls. J. Physiol. 222, 447-474, 1972; Seiffge, D. Methoden zur Untersuchung der Rezeptor-vermittelten Interaktion zwischen Leukozyten und Endothelzellen im Entzündungsgeschehen, in: Ersatz- und Ergänzungsmethoden zu Tierversuchen in der biomedizinischen Forschung, Schöffl, H. et al., (Hrsg.) Springer, 1995 (im Druck)). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspetives of intravital microscopy-bridge over from in vitro to in vivo, Immunology Today 14, 519-522, 1993). Die Applikation der Testsubstanz erfolgt i.v. am Tier (10mg/kg). Die experimentelle Erhöhung der Blutzell-Adhäsion wird durch systemische Verabreichung von Lipopolysaccharid (LPS, 15 mg/kg) 15 Minuten nach Applikation aus Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01.1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigung des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (Angaben in Prozenten).

### Ergebnisse:

Inhibierung der Leukozytenadhäsion [%] (in runden Klammern: Inhibierung des Leukozytenrollings [%]) nach Applikation der Testsubstanz (10mg/kg): Testsubstanz:

| | | |
|---|---|---|
| 7e | 56,2 | (25,7) |
| 7f | 64,6 | (84,7) |
| 7g | 86,9 | (37,8) |
| 7h | 20,8 | (62,8) |

Durchführung von HL60 Zelladhäsionsassays auf rekombinanten, löslichen Adhäsionsmolekülen:
1. 96er Mikrotitertestplatten (Nunc Maxisorb) werden mit 100 µl eines in 50 mM Tris pH 9,5 verdünnten (1+100) Ziege anti human IgG Antikörpers (Sigma) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCl; 1 mg/ml BSA;
   2 mM MgCl₂; 1mM CaCl₂; 3 mM MnCl₂; 0,02 % Natriumazid; 0,2 mM PMSF. Dazu werden 5 µl der Testsubstanz pipettiert, durch Schwenken der Platte vermischt und 10 Min. bei Raumtempeartur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl2; 1 mM CaCl2; 3 mM MnCl2; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

### Ergebnisse:

| Substanz | IC₅₀(µM) für E-Selectin | IC₅₀(µM) für P-Selectin |
|---|---|---|
| 5a | 3.0 | - |
| 5b | 0.4 | 0.6 |
| 5c | 1.5 | 0.9 |
| 18α | 3.1 | 3.1 |
| 18β | 2.4 | 2.5 |
| 19α | - | - |
| 19β | 1.6 | 1.0 |
| 7f | 3.0 | 0.006 |
| 7g | 5.0 | 0.015 |
| 7h | 0.7 | 0.32 |
| 7i | 2.9 | 2.3 |

Die erfindungsgemäßen Verbindungen können demzufolge als Anti-Adhäsionstherapeutika eingesetzt werden und verhindern im Fall von Entzündungen, daß die ELAM-1-Rezeptoren auf stimulierten Endothelzellen an Sialyl Lewis-X-Strukturen auf der Oberfläche von Leukozyten binden. Im Fall der Influenza-Therapie verhindern die Erfindungsgemäßen Verbindungen die Anheftung von Viren an die Neuraminsäure auf der Zelloberfläche und damit auch die Endozytose der Viruspartikel.

Aus diesem Sachverhalt ergibt sich die Möglichkeit zur Prophylase, Diagnostik oder Therapie Selektin-vermittelter Krankheiten. Hierzu gehören beispielsweise:
1. Autoimmunkrankheiten:
   Rheumatische Arthritis, Multiple Sklerose, entzündliche Knochenerkrankungen, Lupus, Myasthenia gravis, Allergien, Osteoarthritis, Asthma, Kontakt-Dermatitis, Psoriasis, Adult respiratory distress syndrome, Transplantatabstoßung.
2. Infektionen:
   Schnupfen, Grippe, Helicobacter pylori, Malaria, Septischer Schock.
3. Krebs:
   Colorectal, Brust, Eierstöcke, Prostata.
4. Zentrales Nervensystem:
   Schlaganfall, Trauma
5. Reperfusionsverletzungen:
   Myocardinfarkt, Angioplastie, instabile Angina, systemischer Schock
6. Weitere:
   Osteroporose, Wunden und schwere Verbrennungen.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche expirmiert werden und demzufolge die zu verabreichende Tagesdosis geringer ist als bei stark erkrankten Patienten.

Die erfindungsgemäßen Arzneimittel werden dadurch hergestellt, daß man eine Verbindung gemäß der vorliegenden Erfindung mit üblichen Träger- sowie gegebenenfalls Zusatz- und/oder Hilfsstoffen in die bzw. eine geeignete Darreichungsform bringt.

Die Verbindungen gemäß der vorliegenden Erfindung sind ferner auch zur Herstellung von Antikörpern zur diagnostischen Bestimmung von Liganden geeignet, welche nicht zugänglich, nicht immunogen genug oder unbekannt sind.

Bei vielen Autoimmunerkrankungen und Tumoren sind bestimmte Liganden bzw. Antigene auf der Zellmembran hochreguliert. Diese sind aber häufig nicht bekannt, lassen sich nicht rein isolieren oder sind nicht genug immunogen, um daraus Antikörper herstellen zu können.

Die Verbindungen gemäß der vorliegenden Erfindung können zur Gewinnung von Antikörpern dienen, welche mit Epitopen der natürlichen, unbekannten bzw. nicht zugänglichen Liganden kreuzreagieren. Neben den diagnostischen Anwendungen sind für die auf diese Weise gewonnenen Antikörper auch therapeutische Anwendungen denkbar (A. N. Houghton, D. A. Scheinberg, Semin. Oncol. 13 (1986) 165-179; W. C. Eckelmann, In Vivo Diagnosis and Therapy of Human Tumors with Monoclonal Antibodies; Pergamon Press, London 1988; M. H. Ravindranath, D. L. Morton, R. F. Irie, Cancer Res. 49 (1989) 3891-3897).

### Spezielle Ausführungsbeispiele

### Verwendete Abkürzungen

- Ac: Acetyl
- All: Allyl
- Bn: Benzyl
- DMF: Dimethylformamid
- DMSO: Dimethylsulfoxid
- DMTr: 4,4'-Dimethoxytrityl
- Hex: n-Hexyl
- NANA: N-Acetylneuraminsäure
- PdC: Palladium auf Aktivkohle
- TBABr: Tetrabutylammoniumbromid
- Tf₂O: Trifluormethansulfonsäureanhydrid
- TMSOTf: Trimethylsilyltrifluormethansulfonat

### Beispiel 1

### a) Synthese von Ethylenglycol-mono-O-2,3,4-tri-O-acetyl-6-O-benzyl-β-D-galactopyranosid (2a)

Zu einer Lösung von O-(2,3,4-Tri-O-acetyl-6-O-benzyl-α/β-D-galactopyranosyl)-trichloracetimidat (1.5 g, 2.77 mmol) und Ethylenglycol (310 µl, 5.55 mmol) in wenig Diethylether/Dichlormethan (1:1) tropft man eine 0.1 M Lösung von TMSOTf (2.8 ml). Nach 30 min wird mit festem Natriumhydrogencarbonat (0.5 g) neutralisiert, filtriert und im Vakuum eingeengt. Nach Flashchromatografie [Toluol/Aceton (4:1)] erhält man Verbindung 2a (890 mg, 73 %). ¹H-NMR (300 MHz, CDCl₃): δ = 1.98, 2.07, 2.08 (3s, 9H, 3OAc), 2.35 (bs, 1H, OH), 5.02 (dd, 1H, 3-H), 5.21 (dd, 1H, 2-H), 5.45 (dd, 1H, 4-H).

### b) Synthese von Ethylenglycol-O-(2,3,4-tri-O-benzyl-α-L-fucopyranosyl)-2,3,4-tri-O-acetyl-6-O-benzyl-β-D-galactopyranosid (3a)

Eine Lösung von 2a (865 mg, 1,96 mmol) wird 1h mit Molekularsieb 4 A, TBABr (218 mg, 968 mmol) und CuBr₂ (1.18 g, 529 mmol) in DMF/Dichlormethan (1:5, 48 ml) gerührt. Dann wird eine Lösung von Thioethyl-O-2,3,4-tri-O-benzyl-β-L-fucopyranosid (1.41 g, 2.94 mmol) in Dichlormethan (2 ml) zugegeben. Nach 24 h wird durch Kieselgur filtriert, mit Dichlormethan nachgespült und mit Natriumhydrogencarbonat gefolgt von Wasser gewaschen. Einengen und Chromatographie (Hexan/Essigester 4:1) liefert 3a (1.3 g, 77 %). ¹H-NMR (300 MHz, CDCl₃):δ=1.08(d, 3H, 6-H_{fuc}), 1.96, 1.99, 2.03 (3s, 9H, 3OAc), 5.16 (dd, 1H, 2-H_{gal}), 5.41 (dd, 1H, 4-H_{gal}).

### c) Synthese von Ethylenglycol-O-(2,3,4-tri-O-benzyl-α-L-fucopyranosyl)-6-O-benzyl-β-D-galactopyranosid (4a)

Eine Lösung von 3a (1.3 g, 1.52 mmol) in Methanol (100 ml) wird mit 1 M Natriummethanolatlösung versetzt. Nach 3 h wird mit Amberlite IR-120 neutralisiert, filtriert und im Vacuum eingeengt. Flaschchromatografie (Dichlormethan/Methanol 20:1) liefert 4a (852 mg, 77 %). ¹H-NMR (300 MHz, CDCl₃):δ=1.10 (d, 3H, 6-H_{fuc}), 2,48, 2.53 (2d, 2H, 2OH).

### d) Synthese von Ethylenglycol-O-(α-L-fucopyranosyl)-(5-acetamido-3,5-didesoxy-D-glycero-α-D-galacto-2-nonulopyranosylsäure)-(2-3)-β-D-galactopyranosid (5a)

Zu einer Mischung aus 4a (832 mg, 1.14 mmol) und Methyl-S-(methyl-5-acetamido-2,4,7,8,9-penta-O-acetyl-3,5-didesoxy-D-glycero-D-galacto-2-nonulopyranosylnat) (891 mg, 1.71 mmol) und Molekularsieb (3A) in Dichlormethan/Acetonitril (18 ml, 5:4) gibt man bei -40° C N-lodsuccinimid (770 mg, 3.42 mmol) und Trifluormethansulfonsäure (30 µl, 0.34 mmol). Nach 2 h wird die tiefrote Lösung mit Natriumhydrogencarbonat (0.5 g) neutralisiert und anschließend filtriert und mit Dichlormethan (100 ml) nachgewaschen. Es wird mit 20 %iger Natriumthiosulfatlösung (100 ml) und Wasser (50 ml) ausgeschüttet, über Natriumsulfat getrocknet, filtriert und eingeengt. Der Rückstand wird in Methanol (40 ml) gelöst und mit 1 M Natriummethanolatlösung versetzt. Nach 4 h wird mit Amberlite IR-120 neutralisiert, filtriert und im Vakuum eingeengt. Die Lactone werden durch Flaschchromatografie (Dichlormethan/Methanol 15:1 → 10:1 → 9:1 → 8:1 → 7:1) isoliert, in Methanol/Dioxan (6:1, 66 ml) aufgenommen, mit Palladiumkohle (10 %, 350 mg) versetzt und 24 h unter eine Wasserstoffatmosphäre hydriert. Es wird filtriert, der Filterkuchen mit Methanol gespült und im Vakuum eingeengt. Der Rückstand wird mit Methanol (25 ml) aufgenommen und mit 1 M Natronlauge (5 ml) versetzt. Neutralisieren mit Amberlite IR-120 und Einengen im Vacuum liefert nach Biogelchromatografie Verbindung 5a (190 mg, 41 %). 1H-NMR (300 MHz, D₂O):δ=1.03 (d, 3H, 6-H_{fuc}), 1.62 (dd, 1H, 3-Hₙₐₙₐ), 1.85 (s, 3H, NAc), 2.58 (dd, 1H, 3-Hₙₐₙₐ), 4.34 (d, 1H, 1-H_{gal}), 4.75 (d, 1H, 1-H_{fuc}).

### Beispiel 2

### a) Synthese von (1R,2R)-trans-1,2-Cyclohexandiol-mono-O-2,3,4-tri-O-acetyl-6-O-benzyl-β-D-galactopyranosid (2b)

Verbindung 2b wird analog zu 2a synthetisiert.

### b) Synthese von (1R,2R)-trans-Cyclohexandiol-O-(2,3,4-tri-O-benzyl-α-L-fucopyranosyl)-2,3,4-tri-O-acetyl-6-O-benzyl-β-D-galactopyranosid (3b)

Verbindung 3b wird analog zu 3a synthetisiert.

### c) Synthese von (1R,2R)-trans-Cyclohexandiol-O-(2,3,4-tri-O-benzyl-α-L-fucopyranosyl)-6-O-benzyl-β-D-galactopyranosid (4b)

Verbindung 4b wird analog zu 4a synthetisiert.

### d) Synthese von (1R,2R)-trans-1,2-Cyclohexandiol-O-(α-L-fucopyranosyl)-(5-acetamido-3,5-didesoxy-D-glycero-α-D-galacto-2-nonulopyranosylsäure)-(2-3)-β-D-galactopyranosid (5b)

Verbindung 5b wird analog zu 5a hergestellt. 1H-NMR (300 MHz, D₂O):δ=0.98 (d, 3H, 6-H_{fuc}), 1.04 (m, 4H, 4-H_{cyclohex}, 5-H_{cyclohex}), 1.51, 193 (2m 4H, 3-H_{cyclohex}, 6-H_{cyclohex}), 1.68 (dd, 1H, 3-Hₙₐₙₐ), 1.84 (s, 3H, NAc), 2.55 (dd, 1H, 3-Hₙₐₙₐ), 3.94 (dd, 1H), 4.38 (d, 1H, 1-H_{gal}), 4.45 (m, 1H, 5-H_{fuc}), 4.79 (d, 1H, 1-H_{fuc}).

### Beispiel 3

### a) Synthese von 1,4-O-n-Hexyl-2,3-O-isopropyliden-D-threitol (6)

Zu einer Mischung aus (-)-2,3-O-Isopropyliden-D-Threitol (5.0 g, 30.8 mmol), Tetrabutylammoniumhydrogensulfat (5.23 g, 15.4 mmol), Toluol (60 ml) und 50 %iger Natronlauge (50 ml) gibt man unter Rühren Bromhexan (13 ml, 92.4 mmol) und nach 24 h weitere 13 ml. Nach insgesamt 48 h werden Wasser (100 ml) und Hexan (200 ml) zugegeben, 10 min gerührt, die organische Phase abgetrennt und mit Wasser (50 ml) gewaschen. Zur organischen Phase gibt man so lange Trockeneis, bis das Waschwasser neutral reagiert. Die organische Phase wird eingeengt und mittels Flashchromatografie (Hexan/Essigester 8:1) gereinigt.

Ausbeute: 8.3 g (81 %). 1H-NMR (300 MHz, CDCl₃):δ=0.89 (2dd, 6H, 2Me), 1.29 (m, 12 H, 6 CH₂), 1.42 (s, 6H, 2Me_{isopropyliden}), 1.58 (m, 4H, 2CH₂), 3.47 (m, 4H, 2CH₂), 3.56 (m, 4H, 2CH₂), 3.97 (m, 2H, 2-H, 3-H).

### b) Synthese von 1,4-O-Hexyl-2,3-D-threitol (7)

Eine Lösung von 6 (8.3 g, 24.96 mmol) in 50 %iger Essigsäure (400 ml) wird 4 h bei 90° C gerührt. Die Mischung wird im Vakuum eingeengt und über eine kurze Kieselgelsäule gereinigt (Toluol/Aceton 5:1). Ausbeute: 7.0 g (96 %).

### c) Synthese von 1,4-O-n-Hexyl-2,3-D-threitol-mono-O-2,3,4-tri-O-acetyl-6-O-benzyl-β-D-galactopyranosid (2c)

Verbindung 2c wird in Analogie zu 2a aus 7 synthetisiert.
¹H-NMR (300 MHz, CDCl₃): δ = 0.88 (m, 6 H, 2 Me), 1.29 (m, 12 H, 6 CH₂), 1.97, 2.04, 2.04 (3 s, 9 H, 3 OAc), 2.88 (d, 1 H, OH), 5.01 (dd, 1 H, 3-H), 5.16 (dd, 1 H, 2-H), 5.46 (dd, 1 H, 4-H).

### d) Synthese von 1,4-O-n-Hexyl-2,3-D-threitol-O-(2,3,4-tri-O-benzyl-α-L-fucopyranosyl)-2,3,4-tri-O-acetyl-O-benzyl-β-D-galactopyranosid (3c)

Verbindung 3c wird analog zu 3a synthetisiert.
¹H-NMR (300 MHz, CDCl₃): δ = 1.08 (d, 3 H, 6-H_{fuc}), 1.96, 1.99, 2.03 (3 s, 9 H, 3 OAc), 5.16 (dd, 1 H, 2-H_{gal}), 5.41 (dd, 1 H, 4-H_{gal}).

### e) Synthese von 1,4-O-n-Hexyl-2,3-D-threitol-O-(2,3,4-tri-O-benzyl-α-L-fucopyranosyl)-6-O-benzyl-β-D-galactopyranosid (4c)

Verbindung 4c wird analog zu Verbindung 4a synthetisiert.

### f) Synthese von 1,4-O-n-Hexyl-2,3-D-threitol-O-(α-L-fucopyranosyl)-(5-acetamido-3,5-didesoxy-D-glycero-α-D-galacto-2-nonulopyranosylsäure)-(2→3)-β-D-galactopyranosid (5c)

Verbindung 5c wird analog zu 5a synthetisiert.
¹H-NMR (300 MHz, D₂O): δ = 0.72 (m, 6 H, 2 Me), 1.04 (d, 3 H, 6-H_{fuc}), 1.15 (m, 12 H, 6 CH₂), 1.40 (m, 4 H, 2 CH₂), 1.74 (dd, 1 H, 3-Hₙₐₙₐ), 1.88 (s, 3 H, NAc), 2.58 (dd, 1 H, 3-Hₙₐₙₐ), 4.38 (d, 1 H, 1-H_{gal}), 4.91 (d, 1 H, 1-H_{fuc}).

### Beispiel 4

### a) Synthese von D-α/β-Isopropyliden-glycerin-γ-allylether (8)

Zu einer Mischung aus DMF (80 ml), Allylbromid (8.18 ml, 96.72 mmol), und Natriumhydrid (2.51 g, 104.78 mmol) gibt man bei 25 bis 30°C D-α/β-Isopropylidenglycerin.

Nach 18 h gibt man Methanol (1.5 ml) und nach 30 min Wasser (500 ml) dazu. Die wäßrige Phase wird mit Ether (3x300 ml) extrahiert und die vereinigten organischen Extrakte mit Wasser (3x300 ml) gewaschen, eingeengt und chromatographiert (Hexan/Essigester 5:1). Man erhält 11.78 g (71 %) 8.

### b) Synthese von (R)-3-Allyloxy-1,2-propandiol (9)

Eine Mischung aus 8 (11.78 g, 68.5 mmol) und 50 %iger Essigsäure (500 ml) wird 3 h lang auf 60°C erwärmt. Anschließend wird im Vakuum eingeengt und 3x mit Toluol nachdestilliert. Man erhält 8.5 g (95 %) 9.

### c) Synthese von (R)-3-Allyloxy-1-Dimethoxytrityloxy-2-propanol (10)

Eine Mischung aus 9 (8.3 g, 63.36 mmol), Pyridin (150 ml) und Dimethoxytritylchlorid (21.5 g, 63.36 mmol) wird 30 min gerührt, eingeengt, der Rückstand in Dichlormethan (300 ml) gelöst und mit 1.2 %iger Natriumhydrogencarbonatlösung (500 ml) sowie Wasser (3x100 ml) gewaschen. Die organische Phase wird eingeengt und chromatographiert (Hexan/Essigester 3:1 + 1 % Triethylamin). Ausbeute: 26.1 g (95 %).

### d) (R)-3-Allyloxy-2-benzyloxy-1-Dimethoxytrityloxy-propan (11)

Zu einer Mischung aus DMF (100 ml), Benzylbromid (10.4 g, 60.83 mmol) und Natriumhydrid (1.58 g, 65.9 mmol) gibt man 10 (22 g, 50.69 mmol). Nach 18 h werden Methanol (10 ml) und nach weiteren 30 min Wasser (400 ml) zugegeben. Es wird mit Ether ertrahiert (3x250 ml) und die vereinigten organischen Extrakte mit Wasser (3x250 ml) gewaschen. Einengen und Flashchromatographie (Hexan/Essigester 7:1 → 5.5:1 → 4:1) liefern 11 (23.47 g, 88 %).

### e) (R)-3-Allyloxy-2-benzyloxy-1-propanol (12)

Verbindung 11 (23.47 g, 44.8 mmol) wird 15 min mit 50 %iger Essigsäure (500 ml) behandelt. Einengen im Vakuum und Chromatographie (Hexan/Essigester 5:1 → 4:1 → 3:1 → 2:1) liefern 12 (8.95 g, 90 %).

### f) Synthese von 3-Allyloxy-2-benzyloxy-1-trifluormethansulfonyloxy-propan 13a

Zu einer Mischung aus Dichlormethan (130 ml) und Pyridin (3.19 g, 40.3 mmol) tropft man bei -30°C Trifluormethansulfonsäure (5.95 ml, 36.3 mmol). Danach wird bei -25°C Verbindung 12 (8.95 g, 40.3 mmol) in Dichlormethan (60 ml) zugetropft. Man läßt auf Raumtemperatur kommen, engt im Vakuum ein und chromatographiert (Hexan/Essigester 6:1). Man erhält 13a (13 g, 91 %).

### g) Synthese von 3-Allyloxy-2-benzyloxy-1-(O-(1R,2R)-trans-1,2-cyclohexandiol)-propanol 14a

Zu einer Mischung aus (1R,2R)-trans-1,2-Cyclohexandiol (1 g, 8.6 mmol) und Tetrabutylammoniumbromid (1.39 g, 4.3 mmol) in Toluol (80 ml) gibt man 50 %ige wäßrige Natronlauge (8 ml) und 13a (3.05 g, 8.6 mmol). Es wird über Nacht gerührt, mit Ether verdünnt, mit Wasser gewaschen und die organische Phase mit Trockeneis neutralisiert. Flashchromatographie (Hexan/Essigester 4:1 → 3:1) liefert 14a (1.8 g, 65 %).
¹H-NMR (300 MHz, CDCl₃): δ = 1.20 (m, 4 H, 5-H_{cyclohex}, 6-H_{cyclohex}), 1.70, 2.0 (2 m, 2x2 H, 3-H_{cyclohex}, 6-H_{cyclohex}), 3.04, 3.42 (2 m, 2x1 H, 1-H_{cyclohex}, 2-H_{cyclohex}), 3.5-3.86 (m, 5 H, 1,2,3-Hₚᵣₒₚₐₙ), 4.0 (m, 2 H, CH₂=CH-CH₂), 4.68 (2 d, 2 H CH₂Bn), 5.2 (m, 2 H, CH₂=CH-CH₂), 5.9 (m, 1 H, CH₂-CH-CH₂), 7.3 (m, 5 H, Ph).

### h) Synthese von 3-Allyloxy-2-benzyloxy-1-O-(1R,2R)-trans-1,2-cyclohexandiol)-(α-tribenzyl-fucopyranosyl)-propanol 15a

Verbindung 15a wird analog zu 3a synthetisiert.

### i) Synthese von 3-Hydroxy-2-benzyloxy-1-O-(1R,2R)-trans-1,2-cyclohexandiol)-(α-tribenzyl-fucopyranosyl)-propanol 16a

Zu einer Lösung von 15a (3.84 g, 5.21 mmol) in Ethanol/Wasser (9:1, 100 ml) gibt man DBU (0.13 ml) und Wilkinson-Katalysator (0.48 g, 0.52 mmol). Die Mischung wird 40 min unter Rückfluß gekocht, eingeengt und über eine kurze Kieselgelsäule (Hexan/Essigester 7:2) chromatographiert. Der Rückstand (3.9 g) wird in Aceton/Wasser (9:1, 20 ml) gelöst und mit Quecksilber(II)-oxid (1.9 g) und einer Lösung von Quecksilber(II)-chlorid (1.9 g, 7.03 mmol) in Aceton/Wasser (9:1, 40 ml) versetzt. Nach 3 h wird das Reaktiongemisch über Kieselgur abgesaugt und mit Chloroform (200 ml) nachgespült. Das Filtrat wird mit 30 %iger Kaliumiodidlösung (3x30 ml) gewaschen. Die organische Phase wird im Vakuum eingeengt. Flashchromatographie (Hexan/Essigester 3:1 → 2:1) liefert 16a (2.74 g, 76 %). ¹H-NMR (300 MHz, CDCl₃): δ = 1.06 (d, 3 H, 6-H_{fuc}), 2.22 (6, 1 H, OH), 4.97 (dd, 1 H, 4-H_{gal}).

### j) Synthese von 3-(Methyl-5-acetamido-2,4,7,8,9-penta-O-acetyl-3,5-didesoxy-D-glycero-α-D-galacto-2-nonulopyranosylonat)-2-benzyloxy-1-O-(1R,2R)-trans-1,2-cyclohexandiol)-(α-tribenzyl-fucopyranosyl)-propanol 17aα und 3-(Methyl-5-acetamido-2,4,7,8,9-penta-O-acetyl-3,5-didesoxy-D-glycero-β-D-galacto-2-nonulopyranosylonat)-2-benzyloxy-1-O-(1R,2R)-trans-1,2-cyclohexandiol)-(β-tribenzyl-fucopyranosyl)-propanol 17aβ

Verbindung 16a (0.4 g, 0.57 mmol) wird wie bei 5a beschrieben in Acetonitril sialyliert. Mitteldruckchromatographie (Dichlormethan/Methanol 80:1 → 50:1) liefert 17aα (233 mg, 35 %) und das entsprechende β-Sialosid 17 aβ (120 mg, 18 %). Bei Verwendung von Dichlormethan als Lösungsmittel und/oder höheren Temperaturen, max. Raumtemperatur, läßt sich der Anteil des β-Glycosids erhöhen.

### k) Synthese von 3-(5-Acetamido-3,5-didesoxy-D-glycero-α-D-galacto-2-nonulopyranosylsäure)-2-benzyloxy-1-O-(1R,2R)-trans-1,2-cyclohexandiol)-(α-tribenzyl-fucopyranosyl)-propanol 18α und 3-(5-Acetamido-3,5-didesoxy-D-glycero-β-D-galacto-2-nonulopyranosylsäure)-2-benzyloxy-1-O-(1R,2R)-trans-1,2-cyclohexandiol)-(β-tribenzyl-fucopyranosyl)-propanol 18β

Die Verbindungen 18α und 18β werden wie bei 5a beschrieben entschützt. 18α: ¹H-NMR (300 MHz, D₂O): δ = 1.04 (d, 3 H, 5-H_{fuc}), 1.60 (dd, 1 H, 3-Hₙₐₙₐ), 1.87 (s, 3 H, NAc), 2.56 (dd, 1 H, 3-Hₙₐₙₐ), 4.88 (d, 1 H, 1-H_{fuc}). 18β: ¹H-NMR (300 MHz, D₂O): δ =1.03 (d, 3 H, 6-H_{fuc}), 1.49 (dd, 1 H, 3-Hₙₐₙₐ), 1.88 (s, 3 H, NAc), 2.23 (dd, 1 H, 3-Hₙₐₙₐ), 4.89 (d, 1 H, 1-H_{fuc}).

### Beispiel 5

### a) Synthese von 3-Allyloxy-1-trifluormethansulfonyloxy-propan 13b

1. Eine Mischung aus 1,3-Propandiol (10 ml, 138 mmol), Allylbromid (7.6 ml, 90 mmol), Kaliumcarbonat (13.8 g, 100 mmol) und Dibenzo-18-Krone-6 (21 mg, 0.6 ml) wird 24 h lang gerührt. Die Mischung wird filtriert, eingeengt und chromatographiert (Hexan/Essigester). ¹H-NMR (300 MHz, CDCl₃): δ = 0.86 (m, 2 H, CH₂), 2.46 (m, 1 H, OH), 3.62 (m, 2 H, CH₂), 3.76 (m, 2 H, CH₂), 3.98 (m, 2 H, CH₂CH=CH₂), 5.20 (m, 2 H, CH₂CH=CH₂), 5.90 (m, 1 H, CH₂CH₂=CH₂).
2. Zu einer Mischung aus Dichlormethan (12 ml) und Pyridin (0.3 ml, 3.7 mmol) tropft man bei -30°C Trimethylsilyl-trifluormethansulfonat (0.54 ml, 3.32 mmol). Danach wird bei -25°C 1-Allyloxy-3-Propanol (430 mg, 3.7 mmol) in Dichlormethan (6 ml) zugetropft. Man läßt auf Raumtemperatur kommen, engt im Vakuum ein und chromatographiert (Hexan/Essigester 6:1). Man erhält 13b (0.56 g, 61 %).

### b) Synthese von 3-Allyloxy-1-O-(1R,2R)-trans-1,2-cyclohexandiol)-propanol 14b Verbindung 14b wird analog zu 14a aus 13b hergestellt.

### c) Synthese von 3-Allyloxy-1-O-(1R,2R)-trans-1,2-cyclohexandiol)-(α-tribenzyl-fucopyranosyl)-propanol 15b

Verbindung 15b wird analog zu 3a synthetisiert.

### d) Synthese von 3-Hydroxy-1-O-(1R,2R)-trans-1,2-cyclohexandiol)-(α-tribenzyl-fucopyranosyl)-propanol 16b

Verbindung 16b wird analog zu 16a synthetisiert.

### e) Synthese von 3-(Methyl-5-acetamido-2,4,7,8,9-penta-O-acetyl-3,5-didesoxy-D-glycero-α-D-galacto-2-nonulopyranosylonat)-1-O-(1R,2R)-trans-1,2-cyclohexandiol)-(α-tribenzyl-fucopyranosyl)-propanol 17bα und 3-(Methyl-5-acetamido-2,4,7,8,9-penta-O-acetyl-3,5-didesoxy-D-glycero-β-D-galacto-2-nonulopyranosylonat)-1-O-(1R,2R)-trans-1,2-cyclohexandiol)-(β-tribenzyl-fucopyranosyl)-propanol 17bβ

Die Verbindungen 17bα und 17bβ werden analog zu 17a bzw. 5a synthetisiert.

### f) Synthese von 3-(5-Acetamido-3,5-didesoxy-D-glycero-α-D-galacto-2-nonulopyranosylonat)-1-O-(1R,2R)-trans-1,2-cyclohexandiol)-(α-tribenzyl-fucopyranosyl)-propanol 19α und 3-(5-Acetamido-3,5-didesoxy-D-glycero-β-D-galacto-2-nonulopyranosylonat)-1-O-(1R,2R)-trans-1,2-cyclohexandiol)-(β-tribenzyl-fucopyranosyl)-propanol 19β

Die Verbindungen 19α und 19β werden analog zu 5a synthetisiert. 18bα: ¹H-NMR (300 MHz, D₂O): δ = 1.05 (d, 3 H, 6-H_{fuc}), 1.65 (dd, 1 H, 3-Hₙₐₙₐ), 1.86 (s, 3 H, NAc), 2.58 (dd, 1 H, 3-Hₙₐₙₐ), 4.86 (d, 1 H, 1-H_{fuc}). 18bβ: ¹H-NMR (300 MHz, D₂O): δ = 1.05 (d, 3 H, 6-H_{fuc}), 1.60 (dd, 1 H, 3-Hₙₐₙₐ), 1.90 (s, 3 H, NAc), 2.25 (dd, 1 H, 3-Hₙₐₙₐ), 4.87 (d, 1 H, 1-H_{fuc}).

### Beispiel 6

Die Synthese der Verbindungen 20α und 20β erfolgt analog zu den Verbindungen 18aα und 19aβ.

### Beispiel 7

### Synthese von 4,6-Isopropyliden-1,2-didesoxyglucose (1e):

Eine Lösung von Tri-O-acetyl-D-glucal (30 g, 110.17 mmol) in Dioxan (400 ml) wird mit Palladiumkohle (10%, 3g) 24 h in einer Wasserstoffatmosphäre hydriert. Die Mischung wird durch Kieselgur filtriert und eingeengt. Zur Abspaltung der Acetylgruppen wird der Rückstand in Methanol (500 ml) aufgenommen und eine 1 M Natriummethanolatlösung (6 ml) zugegeben. Nach 90 min wird mit Amberlite IR-120 neutralisiert, filtriert und im Vakuum eingeengt. Der Rückstand wird mit Toluol (3 x 250 ml) coevaporiert und in DMF (500 ml) aufgenommen. Zu der Lösung gibt man Dimethoxypropan (140 ml, 114,6 mmol) und p-Toluolsulfonsäure (400 mg). Nach 18 h gibt man Triethylamin (3 ml) zu, läßt noch 15 min rühren und engt im Hochvakuum ein. Chromatographie (Toluol/Aceton 4:1) liefert 1d (33 g, 80 %).
¹H-NMR (300 MHz, CDCl₃): δ = 1.41, 1.51 (2s, 6H, 2 CH₃), 1.76 (ddd, 1H, 2-H), 2.0 (ddd, 1H, 2-H), 2.8 (d, 1H, OH), 3.16 (m, 1H, 1-H), 3.46 (dd, 1H, 6-H), 3.53 (m, 1H, 1-H), 3.7 (dd, 1H, 6-H), 3.86 (dd, 1H, 4-H), 3,96 (m, 1H, 5-H).

### Synthese von 4-Allyloxy-1-hydroxybutyl-(1 → 3)-4,6-Isopropyliden-1,2-didesoxyglucose (2e):

2e wird analog zu 2a synthetisiert.

### Synthese von 4-Allyloxy-1-hydroxybutyl-(1 → 3)-1,2-didesoxyglucose (3e):

Zu einer Lösung aus 2a (4.17 g, 14 mmol) in Dichlormethan (340 ml) gibt man 20%ige Trifluoressigsäure (30 ml). Nach 4 h gibt man Toluol (200 ml) dazu und engt auf die Hälfte ein. Man gibt erneut Toluol dazu und engt ein. Der Rückstand wird mit Dichlormethan/Methanol (50:1 40:1 30:1) chromatographiert. Man erhält 3e (3.26 g, 90%).
¹H-NMR (300 MHz, CDCl₃): d = 1.53 (ddd, 1H, 2-H), 1.66 (m, 4H, CH₂CH₂) 2.0 (ddd, 1H, 2-H), 2.9 (bs, 1H, OH), 5.22 (m, 2H, O-CH₂-CH=CH₂), 5.9 (m, 1H, O-CH₂-CH =CH₂).

### Synthese von 4-Phenyl-1-trifuormethansulfonyloxy-butan (4e)

Zu einer eiskalten Lösung von Trifluoromethansulfonsäureanhydrid (3.8 ml, 23 mmol) in Dichlormethan (35 ml) tropft man unter Rühren eine Mischung aus 4-Phenyl-1-butanol (3ml, 20 mmol), Pyridin (1.6 ml, 20 mmol) und Dichlormethan (10 ml). Nach 1 h gibt man Dichlormethan (65 ml) zu und wäscht mit Wasser (3 x 20 ml), trocknet über Magnesiumsulfat und engt bei 25 °C im Vakuum ein. Der Rückstand wird mit Hexan/Essigester 7:1 chromatographiert. Man erhält 4e (3.8 g, 70 %).
¹H-NMR (300 MHz, CDCl₃): d = 1.84 (m, 4 H, -CH₂-CH₂-), 2.67 (t, 2 H, -CH₂-Ph), 4.52 (t, 2H, -CH₂-OTf), 7.24 (m, 5H, Ph).

### Synthese von 4-Allyloxy-1-hydroxybutyl-(13)-[4-phenyl-1hydroxybutyl-(16)]-1,2-didesoxyglucose (5e):

Eine Mischung aus 3e (850 mg, 3.3 mmol), 4d (1.21 g, 4.3 mmol), Kaliumcarbonat (684 mg, 4.95 mmol) und Dibenzo-18-crown-6 (174 mg, 480 µmol) wird 18 h in Toluol (14 ml) gerührt. Zur Aufarbeitung wird filtriert und chromatographiert (Toluol/Aceton 10:1). Ausbeute: 942 mg (73%).
¹H-NMR (300 MHz, CDCl₃): d = 1.53 (ddd, 1H, 2-H), 1.66 (m, 8 H, 4 CH₂), 2.00 (m, 1 H, 2-H), 2.60 (dd, 2H, CH₂Ph), 2.87 (bs, 1H, OH), 3.95 (m, 2H, O-CH₂-CH=CH₂), 5.22 (m, 2H, O-CH₂-CH=CH₂), 5.91 (m, 1H, O-CH₂-CH=CH₂).

### Synthese von 4-Allyloxy-1-hydroxybutyl-(13)-[(2,3,4,-tri-O-benzyl-α-L-fucopyranosyl)-(14)]-[4-phenyl-1-hydroxybutyl-(16)]-1,2-didesoxyglucose (6e):

Die Fucosylierung erfolgt wie bei 3a beschrieben.

### Synthese von 4-Malonyl-1-hydroxybutyl-(1→3]-[(a-L-fucopyranosyl)-(1→4)]-[4-phenyl-1-hydroxybutyl-(1→6)]-1,2-didesoxyglucose (7e):

7e wird analog zu 7a synthetisiert (5 Stufen aus 6e: deallylieren, tosylieren, malonylieren, hydrieren, verseifen).
¹H-NMR (300 MHz, D₂O): d = 1.0 (d, 3H, 6-H_{fuc}), 2.50 (t, 2H, CH₂Ph), 2.90 (t, 2H), 4.20 (q, 1H, 5-H_{fuc}), 4.70 (d, 1H, 1-H_{fuc}).

## Patentansprüche

1. Verbindung der Formel I 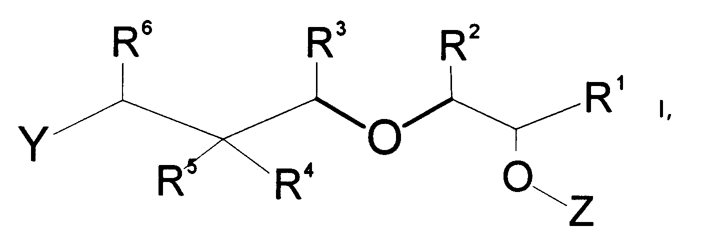 in der
R¹ und R² unabhängig voneinander H, CH₂X oder CH₂O(CH₂)ₘX¹ bedeuten oder gemeinsam einen sechsgliedrigen Carbo- oder Heterocyclus mit mindestens einem der Substituenten R⁷, R⁸ und R⁹ bilden und
R³ O, S, H oder -CH₂OX² bedeutet und
R⁴ und R⁵ unabhängig voneinander O-α-NANA, O-β-NANA, O(CR¹⁰R¹¹)ₙCOOH, OCX³₂(CR¹⁰R¹¹)ₙCOOH, OSO₃H oder eine andere einbasige Säure bedeuten und
R⁶ H, -OH oder C₁-C₂₅-Alkyl bedeutet oder mit R³ einen sechsgliedrigen Carbo- oder Heterocyclus mit mindestens einem Substituenten X⁴ bildet und
Y O-α-NANA, O-β-NANA, O(CR¹⁰R¹¹)ₚCOOH, OCX³₂(CR¹⁰R¹¹)ₚCOOH, OSO₃H oder eine andere einbasige Säure bedeutet und
Z eine Pyranose, eine Furanose oder einen offenkettigen Polyalkohol darstellt und
m, n, p und q unabhängig voneinander eine Zahl von 1 bis 20 und
R⁷, R⁸, R⁹, X, X^{1,} X² und X⁴ unabhängig voneinander H, -NH₂, -COOH, -OH, - CH₂OH, -CH₂NH, C₁-C₂₅-Alkyl, Aryl, oder - CH₂O(CH₂)_{q}X,
X³ H, C₁-C₂₅-Alkyl oder Aryl bedeuten oder wahlweise
X³₂ für =O oder =S steht und
R¹⁰ und R¹¹ unabhängig voneinander X oder -CH₂X bedeuten oder gemeinsam einen sechsgliedrigen Carbo- oder Heterocyclus mit mindestens einem Substituenten X⁴ bilden,
ausgenommen die Verbindungen Sialyl Lewis-X und -A sowie deren Derivate, welche anstelle einer N-Acetylgruppe die Substituenten N₃, NH₂ oder OH oder welche anstelle von Fucose Glycerin tragen.

2. Verbindung nach Anspruch 1, dadurch gekennzeichnet, daß
R³ und R⁶ gemeinsam einen β-D-Galactosylrest bilden.

3. Verbindung nach Anspruch 2, dadurch gekennzeichnet, daß
Z ein O-α-Fucopyranosylrest ist.

4. Verbindung nach Anspruch 3, dadurch gekennzeichnet, daß
Y ein O-α-N-Acetylneuraminsäurerest ist und
R¹ und R² H bedeuten.

5. Verbindung nach Anspruch 3, dadurch gekennzeichnet, daß
Y ein O-α-N-Acetylneuraminsäurerest ist und
R¹ und R² -CH₂O(CH₂)₅CH₃ bedeuten.

6. Verbindung nach Anspruch 3, dadurch gekennzeichnet, daß
R¹ und R² gemeinsam einen sechsgliedrigen Carbocyclus bilden und die Substituenten R⁷, R⁸ und R⁹ H bedeuten.

7. Verbindung nach Anspruch 6, dadurch gekennzeichnet, daß
Y ein O-α-N-Acetylneuraminsäurerest ist.

8. Verbindung nach Anspruch 6, dadurch gekennzeichnet, daß
Y HOOC-CH₂- bedeutet.

9. Verbindung nach Anspruch 2, dadurch gekennzeichnet, daß
R¹ und R² gemeinsam einen sechsgliedrigen Carbocyclus bilden und die Substituenten R⁷, R⁸ und R⁹ H bedeuten, Y ein O-α-N-Acetylneuraminsäurerest ist und Z ein α-Mannosylrest ist.

10. Verbindung nach Anspruch 2, dadurch gekennzeichnet, daß
R¹ und R² gemeinsam einen sechsgliedrigen Carbocyclus bilden und die Substituenten R⁷, R⁸ und R⁹ H bedeuten, Y ein O-α-N-Acetylneuraminsäurerest ist und Z ein α-Glucosylrest ist.

11. Verbindung nach Anspruch 2, dadurch gekennzeichnet, daß
R¹ und R² gemeinsam einen sechsgliedrigen Carbocyclus bilden und die Substituenten R⁷, R⁸ und R⁹ H bedeuten und Z -CH₂C(CH₂OH)₃ bedeutet.

12. Verbindung nach Anspruch 11, dadurch gekennzeichnet, daß
Y ein O-α-N-Acetylneuraminsäurerest ist.

13. Verbindung nach Anspruch 11, dadurch gekennzeichnet, daß
Y ein O-β-N-Acetylneuraminsäurerest ist.

14. Verbindung nach Anspruch 3, dadurch gekennzeichnet, daß
R¹ und R² gemeinsam einen sechsgliedrigen Carbocyclus bilden und die Substituenten R⁷, R⁸ und R⁹ H bedeuten, Y ein O-α-N-Acetylneuraminsäurerest ist und der O-β-D-Galactosylrest in 2-O-Position mit einer Hexylgruppe substituiert ist.

15. Verbindung nach Anspruch 1, dadurch gekennzeichnet, daß
R¹ und R² gemeinsam einen sechsgliedrigen Carbocyclus bilden, R³, R⁶, R⁷, R⁸ und R⁹ H bedeuten und Z ein O-α-Fucopyranosylrest ist.

16. Verbindung nach Anspruch 15, dadurch gekennzeichnet, daß
R⁴ H und R5 -OH bedeuten.

17. Verbindung nach Anspruch 16, dadurch gekennzeichnet,daß
Y O-α-N-Acetylneuraminsäure bedeutet.

18. Verbindung nach Anspruch 16, dadurch gekennzeichnet,daß
Y O-β-N-Acetylneuraminsäure bedeutet.

19. Verbindung nach Anspruch 15, dadurch gekennzeichnet, daß
R⁴ und R5 H bedeuten.

20. Verbindung nach Anspruch 19, dadurch gekennzeichnet, daß
Y α-O-N-Acetylneuraminsäure bedeutet.

21. Verbindung nach Anspruch 19, dadurch gekennzeichnet, daß
Y O-β-N-Acetylneuraminsäure bedeutet.

22. Verbindung nach Anspruch 15, dadurch gekennzeichnet, daß
R⁴ und R5 -CH₂OH bedeuten.

23. Verbindung nach Anspruch 22, dadurch gekennzeichnet, daß
Y O-α-N-Acetylneuraminsäure bedeutet.

24. Verbindung nach Anspruch 22, dadurch gekennzeichnet, daß
Y O-β-N-Acetylneuraminsäure bedeutet.

25. Verbindung nach Anspruch 1, dadurch gekennzeichnet, daß
R¹ und R² gemeinsam einen substituierten Tetrahydropyranring bilden und R⁷ und R⁸ H und
R⁹ -CH₂O(CH₂)_{q}X bedeuten,
Z ein O-α-Fucopyranosylrest ist
R³ und R⁶ gemeinsam einen β-D-Galactosylrest bilden und
Y ein α-N-Acetylneuraminsäurerest ist.

26. Verbindung nach Anspruch 25, dadurch gekennzeichnet, daß
R⁹ -CH₂O(CH₂)₅CH₃ bedeutet.

27. Verbindung nach Anspruch 25, dadurch gekennzeichnet, daß
R⁹ -CH₂OH bedeutet.

28. Verbindung nach Anspruch 25, dadurch gekennzeichnet, daß
R⁹ -CH₂O(CH₂)₃C₆H₅ bedeutet.

29. Verfahren zur Herstellung einer Verbindung der Formel I gemäß einem der Ansprüche 1 bis 28, dadurch gekennzeichnet, daß man zunächst unter Ansprüche 1 bis 28, dadurch gekennzeichnet, daß man zunächst unter Alkylierung oder Glycosylierung einer funktionellen Gruppe eines Akzeptors II; 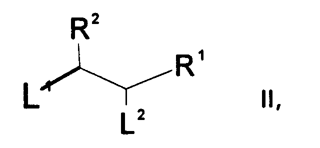 aufweisend mindestens zwei benachbarte funktionelle Gruppen L¹ und L² sowie aufweisend die Substituenten R¹ und R², mittels eines Äquivalentes eines mindestens zwei funktionelle Gruppen L³ und L⁴ tragenden Donors III, 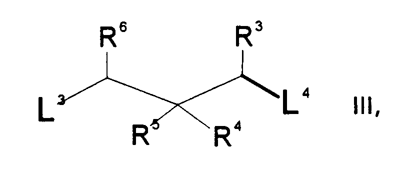 dessen eine funktionelle Gruppe L³ notwendigenfalls geschützt und dessen andere funktionelle Gruppe L⁴ gebenenfalls aktiviert vorliegt, Zwischenverbindung IV, 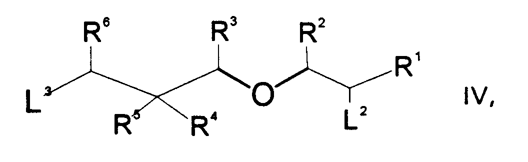 herstellt, wonach man unter Alkylierung, Acylierung oder Glycosylierung der ungeschützten funktionellen Gruppe L² der Zwischenverbindung IV mittels eines mit einer aktivierten funktionellen Gruppe L⁵ versehenen Donors V,
Z-L⁵ V,
dessen übrige funktionelle Gruppen notwendigenfalls Schutzgruppen tragen, Zwischenverbindung VI, 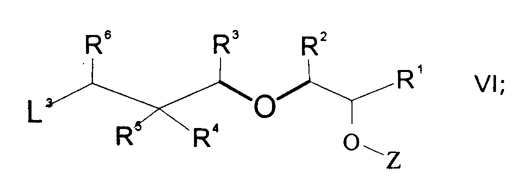 herstellt und schließlich gegebenenfalls nach selektiver Entschützung und Aktivierung der funktionellen Gruppe L³ durch Umsetzung der Zwischenverbindung VI mit Donor VII,
Y-L⁶ VII,
der mit einer aktivierten funktionellen Gruppe L⁶ versehenen und im übrigen mit Schutzgruppen versehen ist, und nachfolgender Abspaltung sämtlicher Schutzgruppen die Verbindung der Formel I gemäß Anspruch 1 erhält, wobei sämtliche übrige Variablen die in Anspruch 1 genannte Bedeutung haben.

30. Verfahren nach Anspruch 29, dadurch gekennzeichnet, daß man Akzeptor II zunächst mit Donor V und anschließend mit Donor III zur Zwischenverbindung VI umsetzt.

31. Arzneimittel, enthaltend eine Verbindung gemäß einem der Ansprüche 1 bis 28 und gegebenfalls pharmazeutische Träger.

32. Verwendung einer Verbindung gemäß einem der Ansprüche 1 bis 28 zur Herstellung eines Arzneimittels zur Therapie oder Prophylaxe von Krankheiten, bei welchen bakterielle oder virale Infektionen, entzündliche Prozesse oder metastasierende Tumoren involviert sind.

33. Verwendung einer Verbindung gemäß einem der Ansprüche 1 bis 28 zur Herstellung eines Mittels zur Diagnose von Krankheiten, bei welchen bakterielle oder virale Infektionen, entzündliche Prozesse oder metastasierende Tumoren involviert sind.
